# EUROPEAN PATENT SPECIFICATION

(11) **EP 3 261 621 B1**
(45) Date of publication and mention of the grant of the patent: **15.10.2025**
(21) Application number: 16705964.1
(22) Date of filing: 24.02.2016
(51) Int. Cl.: A61K 9/14, A61K 9/20, A61K 31/4184, A61P 3/06, A61P 3/10, A61P 9/00, A61P 9/04, A61P 9/10, A61P 9/12, A61P 25/28

(54) **IMMEADIATE RELEASE ORAL TABLET**
ORALE TABLETTE MIT SOFORTIGER FREISETZUNG
COMPRIMÉ ORAL À LIBÉRATION IMMÉDIATE

(30) Priority: 27.02.2015 EP 15156836
(43) Date of publication of application: 03.01.2018
(73) Proprietor: Boehringer Ingelheim International GmbH, 55216 Ingelheim am Rhein (DE)
(72) Inventor: BEHREND, Olaf, 55216 Ingelheim am Rhein (DE); LINKH, Thomas, 55216 Ingelheim am Rhein (DE); NAKATANI, Manabu, 55216 Ingelheim am Rhein (DE)
(74) Representative: Lutze, Oliver
(86) International application number: PCT/EP2016/053825
(87) International publication number: WO 2016/135175

(56) References cited:
- EP-A1- 1 970 053
- WO-A1-2010/012248
- ANONYMOUS: "Handbook of Pharmaceutical Granulation Technology", 2010, CRC PRESS, pages: 389 - 390
- LI YONGCHENG ET AL: "Interactions between drugs and polymers influencing hot melt extrusion", JOURNAL OF PHARMACY AND PHARMACOLOGY : JPP, vol. 66, no. 2, 17 January 2014 (2014-01-17), GB, pages 148 - 166, XP55892771, ISSN: 0022-3573, Retrieved from the Internet <URL:https://watermark.silverchair.com/jphp12183.pdf?token=AQECAHi208BE49Ooan9kkhW_Ercy7Dm3ZL_9Cf3qfKAc485ysgAAAtgwggLUBgkqhkiG9w0BBwagggLFMIICwQIBADCCAroGCSqGSIb3DQEHATAeBglghkgBZQMEAS4wEQQMPAWPVLCjF_ESEzYiAgEQgIICi38TVKUCImRyBam5BzobMgv182wjl68DUWtiPwb-1jxujuF_rFEyq5W_jJyN9fbR90fcZOl323cDLN2m7JEqg5MC-t> DOI: 10.1111/jphp.12183
- M. D. PATIL: "Physicochemical Characterization of Solid Dispersion of Telmisartan with Alkaliser by Hot Melt Method", PHYSICOCHEMICAL CHARACTERIZATION OF SOLID DISPERSION OF TELMISARTAN WITH ALKALISER BY HOT MELT METHOD, 19 March 2013 (2013-03-19), pages 250 - 253, XP093160236, Retrieved from the Internet <URL:https://www.indianjournals.com/ijor.aspx?target=ijor:rjpdft&volume=1&issue=3&article=019>
- VAKA S. R. K. ET AL: "Chapter 4 Excipients for Amorphous Solid Dispersions", AMORPHOUS SOLID DISPERSIONS : THEORY AND PRACTICE, 21 November 2014 (2014-11-21), pages 140 - 145, XP055893484, Retrieved from the Internet <URL:_> [retrieved on 20220218]
- D MANOJ PATIL ET AL: "JPBMS JOURNAL OF PHARMACEUTICAL AND BIOMEDICAL SCIENCES FORMULATION AND EVALUATION OF SOLID DISPERSION OF TELMISARTAN WITH KOH AS ALKALISER BY HOT MELT METHOD", JOURNAL OF PHARMACEUTICAL AND BIOMEDICAL SCIENCES, 1 December 2010 (2010-12-01), pages 1 - 7, XP055186259, Retrieved from the Internet <URL:http://www.jpbms.info/index.php?option=com_docman&task=doc_download&gid=32&Itemid=48.> [retrieved on 20150428]
- P. LEPEK ET AL: "Effect of amorphization method on telmisartan solubility and the tableting process", EUROPEAN JOURNAL OF PHARMACEUTICS AND BIOPHARMACEUTICS, vol. 83, no. 1, 1 January 2013 (2013-01-01), pages 114 - 121, XP055186249, ISSN: 0939-6411, DOI: 10.1016/j.ejpb.2012.09.019
- LIN ZHONG ET AL: "Influence of alkalizers on dissolution properties of telmisartan in solid dispersions prepared by cogrinding", DRUG DEVELOPMENT AND INDUSTRIAL PHARMACY, vol. 40, no. 12, 1 December 2014 (2014-12-01), pages 1660 - 1669, XP055186262, ISSN: 0363-9045, DOI: 10.3109/03639045.2013.841188
- ANONYMOUS: "Lierature Review", 2014, pages 20 - 33, XP002740028, Retrieved from the Internet <URL:http://shodhganga.inflibnet.ac.in/bitstream/10603/28951/9/09_chapter%203.pdf> [retrieved on 20150526]

## Description

The present invention relates to a process for the preparation of a tablet comprising the antihypertensive telmisartan. An immediate release oral tablet produced by the method is disclosed.

### Background of the invention

Telmisartan is an angiotensin II receptor antagonist developed for the treatment of hypertension and other medical indications as disclosed in EP-A-502314. Its chemical name is 4'-[2-n-propyl-4-methyl-6-(1-methylbenzimidazol-2-yl)-benzimidazol-1-ylmethyl]-biphenyl-2-carboxylic acid having the following structure:

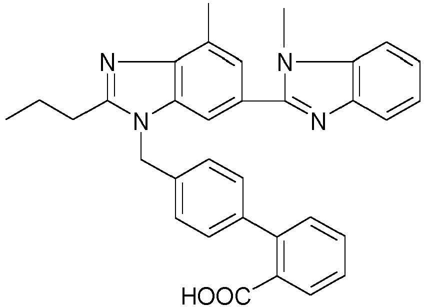

A pharmaceutical tablet or tablet layer comprising telmisartan, a basic agent, and sorbitol was disclosed in EP 1970053A1.

Telmisartan is manufactured and supplied in the free acid form. It is characterized by its very poor solubility in aqueous systems at the physiological pH range of the gastro-intestinal tract between pH 1 to 7. As disclosed in WO 00/43370, crystalline telmisartan exists in two polymorphic forms having different melting points. Under the influence of heat and humidity, the lower melting polymorph B transforms irreversibly into the higher melting polymorph A.

An immediate release (IR) oral tablet is a solid pharmaceutical dosage form, from which the pharmaceutically active agent is rapidly released (in vitro criteria: 75 % dissolved within 45 min according to the European Pharmacopoeia or 85 % within 60 min according to the FDA Guidance for Industry on Dissolution Testing of Immediate Release Solid Oral Dosage Forms).

### Object of the invention

By providing a process based on hot-melt extrusion technology for the preparation of telmisartan tablets with properties of immediate release the present invention facilitates the manufacturing of a pharmaceutical preparation with improved release characteristics of the poorly soluble drug telmisartan compared to current commercial preparations.

### Summary of the invention

The present invention relates to a hot melt extrusion (HME) process for the preparation of a telmisartan tablet. An immediate release HME tablet obtained by such a process is disclosed. The process for the preparation of a telmisartan tablet comprising
(a) processing telmisartan and meglumine by hot melt extrusion at a barrel temperature of 120-180°C to obtain an extrudate of telmisartan;
(b) comminuting the extrudate by milling to obtain a granulate;
(c) blending the granulate with a filler, disintegrant, lubricant and glidant; and
(d) compressing the blend to obtain an immediate release tablet.

### Description of the invention

The present invention refers to a process for the preparation of telmisartan tablets comprising
(a) processing telmisartan, meglumine as the basic agent and optionally a polymeric matrix excipient by hot melt extrusion at a barrel temperature of 120-180°C to obtain an extrudate of telmisartan;
(b) comminuting the extrudate by milling to obtain a granulate and, optionally, split the granules into different particle size fractions by sieving;
(c) blending the granulate with a filler, a disintegrant; lubricant and glidant; and
(d) compressing the blend to obtain an immediate release tablet.

Optionally, the tablet obtained after process step (d) can be coated on the surface.

Unexpectedly, the present invention is able to improve the in-vitro dissolution performance of telmisartan compared to both, the crystalline drug and current commercial Micardis^{®} formulations in dissolution media covering the complete physiologically relevant pH range from strongly acidic gastric conditions (pH 1.2) up to neutral intestinal conditions (pH 6.8).

The active ingredient telmisartan is generally supplied in its free acid form, although pharmaceutically acceptable salts such as the sodium salt may also be used.

The basic agent is meglumine (N-methyl-D-glucamine).

The polymeric matrix excipient of the present disclosure is chosen to form an amorphous solid dispersion with the active agent telmisartan, i.e. telmisartan is embedded into the polymer matrix. Specific examples are polyvinylpyrrolidone, vinylpyrrolidone-vinyl acetate copolymers, polyacrylates, polymethacrylates and cellulose derivatives.

Hot-melt extrusion means processing a powdered premix of an active agent such as telmisartan and functional excipients (e.g. polymers and/or pH modifiers) in an extruder at elevated temperature and mechanical stress to give a pharmaceutical intermediate characterized by a predominantly amorphous, ideally one-phasic structure of the mixture of active agent and excipients. Hot-melt extrusion is preferably performed in heatable twin-screw extruders.

Milling is performed by a cutting mill, hammer mill, ball mill or comparable equipment suitable for comminuting the extruded intermediate of active agent mixed with excipients.

A filler is selected from one or more agents selected from the group consisting of cellulose, lactose, dibasic calcium phosphate anhydrous, erythritol, mannitol, isomalt, pregelatinized starch, microcrystalline cellulose, hydroxypropyl cellulose and other cellulose derivatives. A preferred filler is microcrystalline cellulose or mannitol.

The disintegrant is selected from the group consisting of sodium starch glycolate, crospovidone (cross-linked polyvinylpyrrolidone), corn starch and pregelatinized starch. A preferred disintegrant is crospovidone

Suitable lubricants are sodium stearyl fumarate and magnesium stearate, the latter being preferred.

In process step (a) preferably, 20-70 wt-% of the active agent telmisartan is blended with 10-50 wt-% of the basifier meglumine and, optionally 0-60 wt-% of a matrix polymer. This premix is processed by a twin-screw extruder at barrel temperatures of 120-180 °C to obtain extrusion strands of predominantly amorphous structure. In these, the active agent telmisartan is embedded homogeneously in the excipients, preferably as bi-phasic solid dispersion or, even more preferred, one-phasic solid solution.

Compression of tablets in step (d) is performed with a choice of non-functional tableting excipients, such that fast disintegrating tablets are obtained and the in-vitro dissolution performance of the telmisartan extrudate from process step (a) and the resulting tablets are preferably identical. The fraction of the telmisartan extrudate in the tablet is preferably in a range of 20-80 wt.%, even more preferred 40-60 wt-%.

A further embodiment of the present invention is an immediate release oral tablet comprising:
3 to 50 wt.%, preferably 5 to 30 wt.% of telmisartan;
3 to 30 wt.%, preferably 5 to 15 wt.% of meglumine as the basic agent:
0 to 50 wt.% preferably 0 to 30 wt.% of polymer; and
20 to 92 wt.%, preferably 10 to 80 wt.% of filler
1 to 20 wt%, preferably 1 to 10 wt. % disintegrant
0.5 to 5 wt% preferably 0.5 to 2 wt. % of lubricant and
0.5 to 5 wt%, preferably 0.5 to 2 wt. % of glidant

In particular the disclosed immediate release oral tablet comprises
20-80 mg of the angiotensin II receptor antagonist telmisartan
20-80 mg of the basic excipien meglumine
20-350 mg filler selected from the group consisting of cellulose, dibasic calcium phosphate anhydrous, erythritol, mannitol, microcrystalline
5-50 mg disintegrant selected from the group consisting of sodium starch glycolate, crospovidone, corn starch and pregelatinized starch and
0.01-1.0 mg lubricant such as magnesium stearate.

The tablet contains 10-160 mg amorphous telmisartan, preferably 20-80 mg or 40-80 mg.

Preferred fillers are mannitol, lactose, starch and microcrystalline cellulose;

Preferred disintegrants are crospovidone (cross-linked polyvinylpyrrolidone), corn starch and pregelatinized starch;

Preferred lubricant is magnesium stearate;

Additionally, the tablet can comprise corn starch or pregelatinized starch as a coating agent.

The tablets obtained according to the invention release telmisartan rapidly (e.g. within 30-60 minutes).

To minimize hygroscopicity of the tablets of the present invention they can be packaged using a moisture-proof packaging material such as PVC/PVDC blister in aluminium pouch, aluminium sachet or glass bottles, polypropylene tubes and HDPE bottles which preferably contain a desiccant.

The references to methods of treatment in the subsequent paragraphs of this description are to be interpreted as references to the compounds, pharmaceutical compositions and medicaments of the present invention for use in a method for treatment of the human body by therapy.

A method according to the present invention can be used for the manufacture of an immediate release oral tablet to treat hypertension either alone or in combination with the treatment or prevention of a condition selected from the group consisting of chronic stable angina, vasospastic angina, stroke, myocardial infarction, transient ischemic attack, congestive heart failure, cardiovascular disease, diabetes, insulin resistance, impaired glucose tolerance, pre-diabetes, type 2 diabetes mellitus, diabetic nephropathy, metabolic syndrome (syndrome X), obesity, dyslipidemia, hypertriglyceridemia, elevated serum concentrations of C-reactive protein, elevated serum concentrations of lipoprotein(a), elevated serum concentration of homocysteine, elevated serum concentration of low-density lipoprotein (LDL)-cholesterol, elevated serum concentration of lipoprotein-associated phospholipase (A2), reduced serum concentration of high density lipoprotein (HDL)-cholesterol, reduced serum concentration of HDL(2b)-cholesterol, reduced serum concentration of adiponectin, cognitive decline and dementia.

Particularly preferred is the additional treatment or prevention of chronic stable angina, vasospastic angina, stroke, myocardial infarction, congestive heart failure, diabetes, dyslipidemia or dementia.

In addition to lowering elevated blood pressure (hypertension) the tablet can be used in a method to treat or prevent chronic stable angina, vasospastic angina, stroke, myocardial infarction, congestive heart failure, diabetes, dyslipidemia or dementia.

In order to further illustrate the present invention two telmisartan HME extrudates (see examples 1 and 2) were formulated as 40 mg immediate release HME tablets (see examples 7 and 8). The in-vitro dissolution performance of both HME tablets was tested in comparison to a commercial telmisartan tablet (see examples 12 to 14) in different dissolution media covering in-vivo relevant pH ranges (0.1 M HCl having pH 1.2; Mcllvaine Buffer having pH 4.0; and Mc Ilvaine buffer having pH 6.8). The HME tablets were superior to the commercial tablet under all conditions investigated.

### Examples

### Example 1: Hot Melt Extrusion of Telmisartan

A powder blend containing the following constituents is prepared:

| **Constituents** | **function** | **wt %** |
|---|---|---|
| Telmisartan | active agent | 20.0 |
| Meglumine | basifier | 20.0 |
| Polyvidon VA 64 | matrix polymer | 60.0 |
| **Total** | | **100.0** |

The blend is subsequently processed in a heatable twin-screw extruder. Extrusion is performed at a maximum barrel temperature of 151 °C. Extrusion strands obtained as intermediate product in this process are characterized by a predominantly amorphous structure according to analysis by X-ray powder diffraction and/or differential calorimetry.

### Example 2: Hot Melt Extrusion of Telmisartan

A powder blend containing the following constituents is prepared:

| **Constituents** | **function** | **wt %** |
|---|---|---|
| Telmisartan | active agent | 50.0 |
| Meglumine | basifier | 50.0 |
| **Total** | | **100.0** |

The blend is subsequently processed in a heatable twin-screw extruder. Extrusion is performed at a maximum barrel temperature of 142 °C. Extrusion strands obtained as intermediate product in this process are characterized by a predominantly amorphous structure according to analysis by X-ray powder diffraction and/or differential calorimetry.

### Example 3: Hot Melt Extrusion of Telmisartan

A powder blend containing the following constituents is prepared:

| **Constituents** | **function** | **wt %** |
|---|---|---|
| Telmisartan | active agent | 60.0 |
| Meglumine | basifier | 20.0 |
| Eudragit E PO | matrix polymer | 20.0 |
| **Total** | | **100.0** |

The blend is subsequently processed in a heatable twin-screw extruder. Extrusion is performed at a maximum barrel temperature of 168 °C. Extrusion strands obtained as intermediate product in this process are characterized by a predominantly amorphous structure according to analysis by X-ray powder diffraction and/or differential calorimetry.

### Example 4: Hot Melt Extrusion of Telmisartan

A powder blend containing the following constituents is prepared:

| **Constituents** | **function** | **wt %** |
|---|---|---|
| Telmisartan | active agent | 60.0 |
| Meglumine | basifier | 20.0 |
| Poloxamer 188 | matrix polymer | 20.0 |
| **Total** | | **100.0** |

The blend is subsequently processed in a heatable twin-screw extruder. Extrusion is performed at a maximum barrel temperature of 149°C. Extrusion strands obtained as intermediate product in this process are characterized by a predominantly amorphous structure according to analysis by X-ray powder diffraction and/or differential calorimetry.

### Example 5: Milling of the telmisartan extrudate

The extrusion strands obtained in the hot-melt extrusion (HME) process exhibit a typical diameter of 0.5-2 mm and length of 10-50 mm. To allow manufacturing of tablets with the required content uniformity, this intermediate product is comminuted by means of a cutting mill. Granule size of the comminuted extrudate is less than 630 µm with a calculated d50-value of 200-400 µm according to sieve analysis.

### Example 6: Tableting

Intermediate granules are mixed with different excipients according to the following table and the blend is subsequently compressed to tablets by means of a single punch or rotary tablet press.

| **Constituents** | **function** | **wt %** |
|---|---|---|
| HME granules | active agent | 40.0 |
| MCC grade 101 | binder | 28.0 |
| MCC grade 200 | binder | 28.0 |
| crospovidone | disintegrant | 3.0 |
| silicon dioxide | glidant | 0.5 |
| magnesium stearate | lubricant | 0.5 |
| **Total** | | **100.0** |

### Example 7: 40 mg Telmisartan immediate release tablet

The tablet is described by the following composition:

| **Constituents** | **mg per tablet** | **wt % per tablet** |
|---|---|---|
| Telmisartan | 40.0 | 8.0 |
| Meglumine | 40.0 | 8.0 |
| Polyvidon VA 64 | 120.0 | 24.0 |
| Microcrystalline Cellulose, grade 101 | 140.0 | 28.0 |
| Microcrystalline Cellulose, grade 200 | 140.0 | 28.0 |
| crospovidone | 15.0 | 3.0 |
| silicon dioxide | 2.5 | 0.5 |
| magnesium stearate | 2.5 | 0.5 |
| **Total** | **500.0** | **100.0** |

### Example 8: 40 mg Telmisartan immediate release tablet

The tablet is described by the following composition:

| **Constituents** | **mg per tablet** | **wt % per tablet** |
|---|---|---|
| Telmisartan | 40.0 | 20.0 |
| Meglumine | 40.0 | 20.0 |
| Microcrystalline Cellulose, grade 101 | 56.0 | 28.0 |
| Microcrystalline Cellulose, grade 200 | 56.0 | 28.0 |
| crospovidone | 6.0 | 3.0 |
| silicon dioxide | 1.0 | 0.5 |
| magnesium stearate | 1.0 | 0.5 |
| **Total** | **200.0** | **100.0** |

### Example 9: 20 mg Telmisartan immediate release tablet

The tablet is described by the following composition:

| **Constituents** | **mg per tablet** | **wt % per tablet** |
|---|---|---|
| Telmisartan | 20.00 | 8.0 |
| Meglumine | 20.00 | 8.0 |
| Polyvidon VA 64 | 60.00 | 24.0 |
| Microcrystalline Cellulose, grade 101 | 70.00 | 28.0 |
| Microcrystalline Cellulose, grade 200 | 70.00 | 28.0 |
| crospovidone | 7.50 | 3.0 |
| silicon dioxide | 1.25 | 0.5 |
| magnesium stearate | 1.25 | 0.5 |
| **Total** | **250.0** | **100.0** |

### Example 10: 80 mg Telmisartan immediate release tablet

The tablet is described by the following composition:

| **Constituents** | **mg per tablet** | **wt % per tablet** |
|---|---|---|
| Telmisartan | 80.0 | 8.0 |
| Meglumine | 80.0 | 8.0 |
| Polyvidon VA 64 | 240.0 | 24.0 |
| Microcrystalline Cellulose, grade 101 | 280.0 | 28.0 |
| Microcrystalline Cellulose, grade 200 | 280.0 | 28.0 |
| crospovidone | 30.0 | 3.0 |
| silicon dioxide | 5.0 | 0.5 |
| magnesium stearate | 5.0 | 0.5 |
| **Total** | **1000.0** | **100.0** |

### Example 11: 80 mg Telmisartan immediate release tablet

The tablet is described by the following composition:

| **Constituents** | **mg per tablet** | **wt % per tablet** |
|---|---|---|
| Telmisartan | 80.0 | 20.0 |
| Meglumine | 80.0 | 20.0 |
| Microcrystalline Cellulose, grade 101 | 112.0 | 28.0 |
| Microcrystalline Cellulose, grade 200 | 112.0 | 28.0 |
| crospovidone | 12.0 | 3.0 |
| silicon dioxide | 2.0 | 0.5 |
| magnesium stearate | 2.0 | 0.5 |
| **Total** | **400.0** | **100.0** |

### Example 12: In-vitro dissolution performance at pH 1.2:

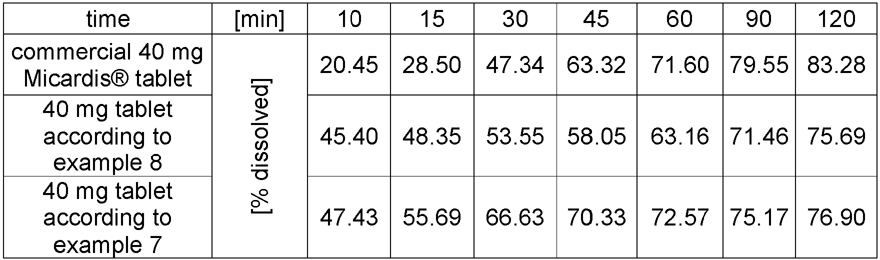

Dissolution testing was performed applying a device similar to USP apparatus 2, at a paddle speed of 50 rpm, using 200 mL of 0.1 M HCl at 37 °C.

### Example 13: In-vitro dissolution performance at pH 4.0:

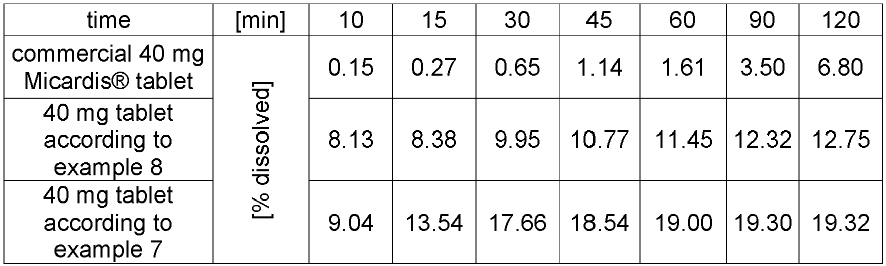

Dissolution testing was performed applying a device similar to USP apparatus 2, at a paddle speed of 50 rpm, using 200 mL of Mcllvaine buffer pH 4.0 at 37 °C.

### Example 14: In-vitro dissolution performance at pH 6.8:

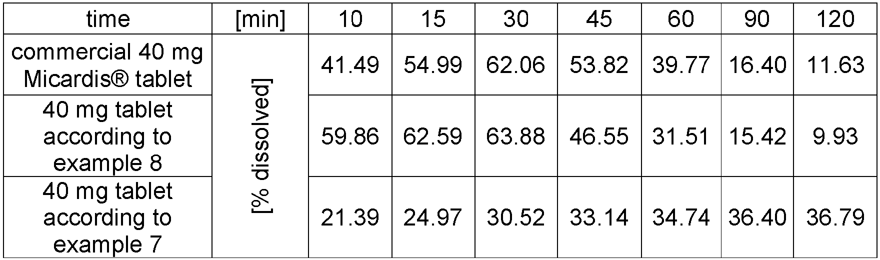

Dissolution testing was performed applying a device similar to USP apparatus 2, at a paddle speed of 50 rpm, using 200 mL of Mcllvaine buffer pH 6.8 at 37 °C.

## Claims

1. A process for the preparation of a telmisartan tablet comprising
(a) processing telmisartan and meglumine by hot melt extrusion at a barrel temperature of 120-180°C to obtain an extrudate of telmisartan;
(b) comminuting the extrudate by milling to obtain a granulate;
(c) blending the granulate with a filler, disintegrant, lubricant and glidant; and
(d) compressing the blend to obtain an immediate release tablet.

2. The process according to claim 1 wherein the filler is selected from the group consisting of cellulose, dibasic calcium phosphate anhydrous, erythritol, mannitol, microcrystalline cellulose, and pregelatinized starch.

3. The process according to claim 1 wherein the tablet obtained in process step (d) is coated on the surface preferably with corn starch or pregelatinized starch.

4. The process according to claim 1 wherein the disintegrant is selected from the group consisting of sodium starch glycolate, crospovidone (cross-linked polyvinylpyrrolidone), corn starch and pregelatinized starch.

5. The process according to claim 4 wherein the disintegrant is crospovidone.

6. The process according to claim 1 wherein the lubricant is selected from the group consisting of sodium stearyl fumarate and magnesium stearate.

7. The process according to claim 6 wherein the lubricant is magnesium stearate.

8. The process according to claims 1 to 4 and 6, wherein the granules obtained after milling in process step (b) are divided into different particle size fractions by sieving.

## Patentansprüche

1. Verfahren zur Herstellung einer Telmisartan-Tablette, umfassend
(a) Verarbeiten von Telmisartan und Meglumin durch Heißschmelzextrusion bei einer Zylindertemperatur von 120 bis 180 °C, um ein Extrudat von Telmisartan zu erhalten;
(b) Zerkleinern des Extrudats durch Mahlen, um ein Granulat zu erhalten;
(c) Mischen des Granulats mit einem Füllstoff, einem Sprengmittel, einem Schmiermittel und einem Fließhilfsmittel; und
(d) Pressen der Mischung, um eine sofort freisetzende Tablette zu erhalten.

2. Verfahren nach Anspruch 1, wobei der Füllstoff ausgewählt ist aus der Gruppe, bestehend aus Cellulose, wasserfreiem dibasischem Calciumphosphat, Erythritol, Mannitol, mikrokristalliner Cellulose und vorgelatinierter Stärke bzw. Quellstärke.

3. Verfahren nach Anspruch 1, wobei die erhaltene Tablette in Verfahrensschritt (d) auf der Oberfläche beschichtet ist, bevorzugt mit Maisstärke oder vorgelatinierter Stärke bzw. Quellstärke.

4. Verfahren nach Anspruch 1, wobei das Sprengmittel ausgewählt ist aus der Gruppe, bestehend aus Natriumstärkeglykolat, Crospovidon (vernetztem Polyvinylpyrrolidon), Maisstärke und vorgelatinierter Stärke bzw. Quellstärke.

5. Verfahren nach Anspruch 4, wobei das Sprengmittel Crospovidon darstellt.

6. Verfahren nach Anspruch 1, wobei das Schmiermittel ausgewählt ist aus der Gruppe, bestehend aus Natriumstearylfumarat und Magnesiumstearat.

7. Verfahren nach Anspruch 6, wobei das Schmiermittel Magnesiumstearat darstellt.

8. Verfahren nach den Ansprüchen 1 bis 4 und 6, wobei die erhaltenen Granulate nach dem Mahlen in Verfahrensschritt (b) durch Sieben in verschiedene Partikelgrößenfraktionen aufgeteilt werden.

## Revendications

1. Procédé de préparation d'un comprimé de telmisartan comprenant
(a) le traitement du telmisartan et de la méglumine par extrusion par fusion à chaud à une température de fût de 120 à 180 °C pour obtenir un extrudat de telmisartan ;
(b) le concassage de l'extrudat par broyage pour obtenir un granulat ;
(c) le mélange du granulat avec une charge, un désintégrant, un lubrifiant et un agent de glissement ; et
(d) la compression du mélange pour obtenir un comprimé à libération immédiate.

2. Procédé selon la revendication 1, dans lequel la charge est choisie dans le groupe consistant en la cellulose, le phosphate de calcium dibasique anhydre, l'érythritol, le mannitol, la cellulose microcristalline et l'amidon prégélatinisé.

3. Procédé selon la revendication 1, dans lequel le comprimé obtenu à l'étape de procédé (d) est enduit sur la surface de préférence d'amidon de maïs ou d'amidon prégélatinisé.

4. Procédé selon la revendication 1, dans lequel le désintégrant est choisi dans le groupe consistant en le glycolate d'amidon sodique, la crospovidone (polyvinylpyrrolidone réticulée), l'amidon de maïs et l'amidon prégélatinisé.

5. Procédé selon la revendication 4, dans lequel le désintégrant est la crospovidone.

6. Procédé selon la revendication 1, dans lequel le lubrifiant est choisi dans le groupe constitué du fumarate de stéaryle de sodium et du stéarate de magnésium.

7. Procédé selon la revendication 6, dans lequel le lubrifiant est du stéarate de magnésium.

8. Procédé selon les revendications 1 à 4 et 6, dans lequel les granulés obtenus après broyage dans l'étape de procédé (b) sont divisés en différentes fractions granulométriques par tamisage.
